# EUROPEAN PATENT APPLICATION

(11) **EP 2 737 894 A1**
(43) Date of publication of application: **04.06.2014**
(21) Application number: 12195245.1
(22) Date of filing: 03.12.2012
(51) Int. Cl.: A61K 9/00, A61L 27/34

(54) **Smart coating for implantable devices**

(71) Applicant: Debiotech S.A., 1004 Lausanne (CH)
(72) Inventor: Piveteau, Laurent-Dominique, 1030 Bussigny (CH); Neftel, Frédéric, 1005 Lausanne (CH)
(74) Representative: Grosfillier, Philippe

(57) **Abstract**

The present invention relates to a coating for medical implants. The coating contains a drug that is intended to have an effect on the surrounding tissue after placement of the implant. This effect is however activated only in the presence of the cells of the targeted tissue. The drug compound is released from the coating under the direct influence of the cells.

## Description

### Field of invention

The present invention relates to a coating for medical implants.

### State of the Art

The standard therapy used to treat the blockage of a coronary artery includes the placement into the (partially) obstructed artery of a stent, a small metallic mesh which is intended to maintain the vessel open after the intervention. It is positioned into the artery with a metallic guide and, once in place, is propped open thanks to a small balloon. One of the most common issues following this type of intervention is restenosis: a post operatory re-closing of the vessel. This occurs in the weeks following the intervention and is due to the anarchical growth of smooth muscle cells (SMC) induced by the trauma of the procedure. In order to limit this occurrence, a majority of stents today is covered with a drug layer (most often integrated into a polymeric structure) which is released over time and acts as a cytotoxic agent against the SMC, therefore blocking their development. This elution process, that takes a few weeks, is controlled in different ways depending on the stent platform: the drug can be loaded in a reservoir-like structure and be released by diffusion through a porous membrane; it may be embedded into a porous membrane and diffuse through the membrane; it may be dissolved into a polymer that will erode over time; or it may simply be attached at the surface of the stent in which case the elution rate is controlled by its own solubility or by the solubility of the link that attaches it to the substrate.

One of the issues that has been raised with most of these coating is the long term toxicity of the material used to store the drug. Different solutions to this problem have been proposed. In WO 03/090818, the coating containing the drug will dissolve over time leaving a pure bare metal stent in the vessel a few months after implantation. In WO 2010/136848 and WO 2007/148240 coatings made of a nanoporous layer containing buried micrometric reservoirs are described. In particular this type of coating is manufactured using ceramics, with the objective of reducing the long term drawbacks of commonly used polymeric materials.

All these systems remain fully passive. Once placed into a solution, the drug present into the coating immediately starts to be released. The elution profile is defined by various physicochemical parameters such as the concentration of the drug in the coating, its solubility, or the type of matrix containing the molecule. It is never initiated of driven by the real need, which is the presence of the cells to be eliminated or treated.

Other systems are using an intermediary signalling system. In D. Koley et al. "Toward a smart catheter that senses microbial attachment and turns on electrochemical release of bactericidal nitric oxide" ACS Meeting 2012, Philadelphia, August 19-23 2012, a coating is activated following a change of pH in the environment. This change of pH, which may be induced by the presence of bacteria and the subsequent reaction of the environment, will activate the coating that will release a bacteriostatic agent. This type of device allows a more specific and targeted response than purely passive systems. However it does not offer the level of efficacy and safety proposed in this application. In particular, the change of pH is not specific to the presence of bacteria and other causes may induce a non-desired release of the drug.

### General Description of the Invention

The present invention relates to a coating for an implantable medical device comprising a structural material and an active agent, said structural material and/or active agent containing a substance which, when brought into contact with a cell, induces the dissolution of said structural material and thereby allows the release of said active agent.

In other words it relates to a coating for a medical device characterized in that:
- the coating dissolves when put in contact with living cells
- the dissolution of the coating is initiated by the cells
- the coating contains an active agent which is released consequently to this dissolution
- the coating prevents the release of the active agent until the cells are brought in contact with the coating.
This invention relates to the creation of a layer that can be coated onto an implant and that will react and release the active agent it contains in the presence, and only in the presence, of cells. This implant may be a stable implant that will remain in place after the dissolution of the coating. It may also be made out of degradable material and be resorbed over time after the dissolution of the coating. The coating itself may be made of different sub-coatings made of different materials. Sub-coating may differ by their structural material, their active agent and / or their substance inducing dissolution.

Coatings offer the advantage of modifying the surface properties of implantable devices while keeping unchanged most of the substrate properties such as for example its mechanical behaviour. In a possible embodiment the coating is dimensioned in order to be fully dissolved when the action of the active agent (in particular a drug) isn't needed anymore. This is of particular interest, as a common problem with polymeric coating is their very poor long term biocompatibility. The release of small particles or chemical by-products (resulting in particular from the ageing of polymers) has been often described as a possible origin of toxicity or carcinogenic effects. The use of a material that disappears completely in the hours, days, weeks or months following the implantation should completely cancel this risk.

The coating in this invention contains an active agent. This agent is intended to be active after the implantation of the device and act on the surrounding cells or tissue. Contrary to other drug eluting coatings described extensively in the literature, it is supposed to be activated by cells present in the environment or cells present into the surrounding tissue and in a preferred embodiment by the targeted cells themselves or by the cells present in the targeted tissue. The active agent will only be released after the coating has been put in contact with a particular tissue.

In a preferred embodiment, the dissolution of the coating will be initiated by defined and limited cell types. Ideally only a single cell type shall be able to initiate the degradation of a given coating. In that sense the coating is specific to a given cell-type. In a possible approach, the coating will react with an enzyme synthetized by, or present at the surface of a specific targeted cell. In other words, the coating will be made of an enzyme-sensitive biomaterial. This approach offers several advantages. First, due to the remarkable substrate selectivity of enzymes and the large database of identified enzyme, a specific coating can potentially be developed for each cell type. Then, as the expression and activity of enzymes may, for a given cell, be closely linked with their general condition (a cell in an inflamed or diseased tissue will express different enzymes than the same cell under normal conditions), it becomes possible to finely tailor the response of the coating to the surrounding tissue: a given cell-type will activate the coating only when certain physiological conditions are met.

In a preferred embodiment, the dissolution of the coating will only take place in presence of the cells. If for any reason the target cells aren't anymore in contact with the tissue, the dissolution process will stop. Some active agents loaded in coatings may sometime be very toxic. They are meant for a given cell-type but are also often detrimental to other cells that may be present in the surroundings. It is therefore preferable to only release the active agent when needed, i.e. in the presence of the targeted tissue. In that sense the coating becomes really specific and minimizes side-effects as it releases its content in the presence of the target cells and only in their presence.

The active agent loaded into the coating may have different functionalities. In the case of a coating for drug eluting stents, it may be decided to use a toxic agent. This may be a drug such as for example paclitaxel, sirolimus, everolimus, tacrolimus, zotarolimus or biolimus, agents which are commonly used today. For orthopaedic implants, where a recurrent problem is post-operative infection, the drug may be chosen in the group of bacteriostatic compounds. In other applications, where an accelerated growth of the surrounding tissue may be desirable, agents favouring cell proliferation may be selected. Another non limiting example is the integration of a contrast medium in the coating. This compound will allow a non-invasive analysis of the tissue reaction after implantation.

In another embodiment, the active agent loaded into the coating may be composed of different molecules. These may be different molecules with the same functionality, such as different bacteriostatic elements offering a broader activity spectrum. This may also be molecules having different functionalities: a combination of a cytotoxic drug and a contrast medium, for example. In that case it would become possible to diagnose and image a reaction of the surrounding tissue and treat it at the same time.

It is interesting to note here the potency of this approach in the case of bacterial infection. With this type of approach, it is possible to design a coating that will react and only react to a specific type of bacteria. An active agent such as an antibiotic that will be loaded in the coating will be especially targeted toward these bacteria and chosen for its efficacy. In the presence of the bacteria, it will be released locally with high efficiency. The antibiotic will however not be released if the targeted bacteria is not present and this will therefore limit the risk of generating resistant strains.

Another example of the potency of this approach is the possibility to give an advanced response to a complex cellular process. For example, the vascular injury following the placement of a stent in an artery will result in a series of events that can roughly be categorized into thrombosis, inflammation, proliferation of the SMC in the intima and media of the artery, migration of the medial SMC into the intima, and secretion of an extracellular matrix. By placing at the surface of the stent different coatings reacting to the presence of different cells (or to the same cell but under different physiological conditions), it becomes possible to release different drugs depending of the event currently occurring and encouraging or, on the contrary, inhibiting such events.

This coating also allows a temporal and spatial control of the drug release. This release is additionally initiated and controlled by the targeted cells. It therefore allows a smart and efficient answer with limited secondary effects.

The coating may be made out of various materials but in a preferred embodiment it will be made of a polymeric material, and more precisely of a hydrogel. A typical coating will contain at least two elements: a polymer structure and cell-sensitive spacers. The polymer will ideally be non-soluble and biocompatible. For example it may be made of multi-branched PEG chains or dendrimer molecules. Multi-branching is quite favourable to the creation of the mesh-type, porous structure of the coating. The spacer will ideally be an enzyme-sensitive entity. In the absence of the targeted cells, the coating is stable. The size of the porosity is chosen so that active agent molecules cannot diffuse into the surrounding tissue. In the presence of the target cell, the spacer will be cleaved, destroying the mesh structure of the coating and liberating the active compound.

In a preferred embodiment, the spacer will be made of a series of peptides. These peptides will be cleaved by enzymes secreted or present at the surface of the target cells. The combinations peptide-enzyme is very potent due to its diversity and specificity. The literature gives a lot of examples of peptide sequences - enzyme couples. Typical enzymes that have been studied are for example azoreductase, tyrosine kinase, cathepsins, alkaline and acid phosphatase, matrix metalloproteinases, elastase, lipase, β-lactamase, thermolysin, plasmin, collagenase, urokinase, plasminogen-activator or factor Xa.

In some cases it may be of interest to additionally link the active agent to the matrix of the coating and in particular to the polymer part of the coating. This may be the case for small molecules that could be smaller than the size of the porosity of the coating. If these molecules were not attached, they could be released simply by diffusion through the structure. The link between the active agent and the coating may be soluble or stable. A soluble link will be preferred for ingredient that may lose their activity (or see it dramatically reduced) if linked to a large molecule. The table below gives different possible approach as a function of the relatives sizes of the active ingredient, the activating molecule such as for example the enzyme expressed by the cell and the porosity of the coating.

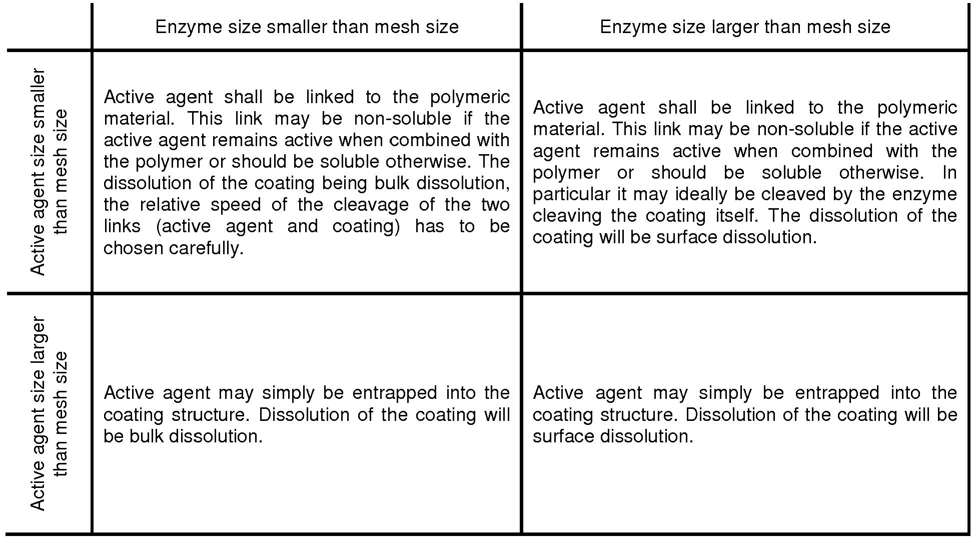

In a possible embodiment, the soluble link between the active agent and the coating may be an enzyme reactive molecule. In the case of a coating where dissolution is initiated by an enzyme, the link may react to the same enzyme or it may react to a different enzyme. In the case where the enzyme used to dissolve the coating differs for the enzyme used to detach the drug molecule, both reaction may be induced by the same cell (in which case this cell expresses both enzymes) or by distinct cells.

Here again the active agent present in the coating doesn't have to be limited to a single molecule. Several different molecules having similar or very different effect on the surrounding tissue may be linked to the coating through a soluble or non-soluble link. As above, the parameters driving the dissolution of the coating and the potential cleavage of the link between the active agent and the coating have to be chosen according to the specific application.

As mentioned in the table above, if the size of the active agent is larger than the size of the porosity of the coating, the active agent may simply be entrapped into the coating structure. It may be dissolved or precipitated into the coating. In may also, in another embodiment, be further encapsulated and the capsules may themselves be entrapped into the coating. The material of these capsules may further be degradable. It may also be attached to a carrier: polymer chains, branched polymer chains, dendrimers, particles...

A known drawback of coated materials is the weakness of the mechanical interface between the substrate and the coating itself. Most of the time, coating and substrate materials are different and adhesion is purely mechanical. The roughness of the substrate acts as an anchoring structure to which the coating will adhere. The presence of a chemical link between the coating and the substrate (in addition to the mechanical link) will increase the quality of the interface and improve its resistance to wear, tear or fatigue. It may therefore be preferable in some embodiments to use an adapted chemistry to create (in addition to a mechanical link) a chemical link between the substrate and the coating. This chemical link may take the form of an intermediary layer such as poly-(L-lysine)-g-poly-(ethylene glycol) (PLL-g-PEG) grafted co-polymers.

### Figures

Figure 1 shows a first possible embodiment of the invention where the target cell cleaves the peptide links to dissolve the coating and, as a consequence, liberate the active ingredient.
Figure 2 shows another possible embodiment of the innovation where the target cell first cleaves the peptide links (dissolving the coating) to access the active agent and then cleaves the link between the active agent and the coating.

### Detailed Description of the Invention

For the sake of simplicity and of clarity, in the detailed description of the invention we will concentrate on coatings made of a polymeric substance, where the dissolution is induced using an enzymatic reaction. This invention however encompasses coatings made out of other materials such as for example metals or ceramics and involving other mechanisms to trigger the dissolution.

Under normal conditions, enzymes are tightly regulated to coordinate biochemical and biophysical activities necessary to sustain life. Enzyme expression is often related to cell activity. Enzymes and diseases are closely associated therefore the expression and activity of enzymes are good indicators of the progression and prognosis of a disease. Linking the activity of a coating to the expression of an enzyme is therefore very interesting. Their multitude (several thousands of enzymes have been identified to date), their very high specificity towards a predefined substrate and their link to different physiological states of a living tissue make them very interesting candidates as activators of smart drug eluting coatings.

For example, in the case of drug eluting stents, the smooth muscles cells present after the stenting express large amounts of MMP-2 and MMP-9 proteases. Johnson and Gallis (in C. Johnson and Z. Galis, "Matrix Metalloproteinase-2 and -9 Differentially Regulate Smooth Muscle Cell Migration and Cell-Mediated Collagen Organization", Arterioscler. Thromb. Vasc. Biol., 24 (2004) 54-60) have demonstrated that hyperplasia conducting to restenosis which is observed after stenting could be strongly reduced in mice where the genes governing the synthesis of MMP-2 and MMP-9 has been knocked out. On the contrary, an over-expression of MMP-9 in the carotid of a rat model generated a higher migration of SMC and a more intense remodelling of the tissue. By choosing a material sensitive to MMP-2 or MMP-9, it becomes possible to specifically act in the presence of SMC involved in the restenosis process. A coating liberating at this point of time a toxic substance against these cells would prevent them to proliferate anarchically and, as a consequence, block the lumen of the artery.

A typical coating described in this invention is made up of three elements:
- a polymer structure,
- an enzyme sensitive cross-linking agent placed between the chains of the polymer and
- active molecules such as for example drug.

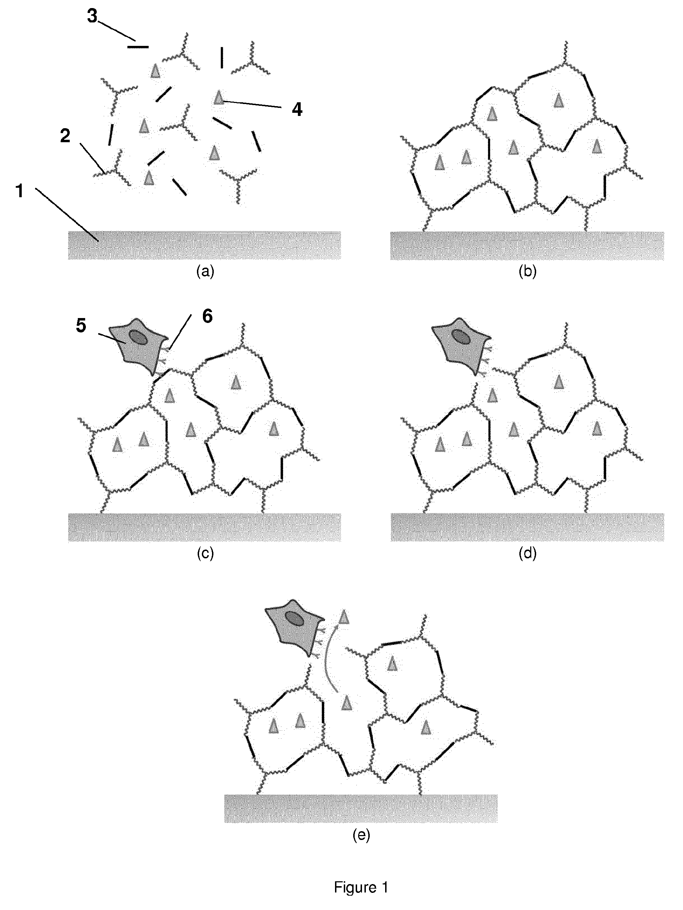

The figure 1(a) shows these different elements before there combination to create a coating: multi-branched polymer chains (2), peptide cross-linkers (3) and drug molecules (4). On a surface (see figure 1(b)) the multi-branched polymer chains (2) and the peptide cross-linkers are combined to form a three dimensional network. During the formation process, the drug molecules (4) are trapped into the network. In the presence of a cell (5) (figure 1(c)) expressing a predetermined enzyme (6) peptide cross-linkers will be cleaved (figure 1(d)) creating openings into the network. This will allow the entrapped drug molecules (4) to be liberated (figure 1(e)). This process will continue as long as cells expressing the enzyme cleaving the cross-linking peptide will be present. With time, polymer chains will be liberated and cleared by the environment; the coating will be dissolved and the drug eluted.

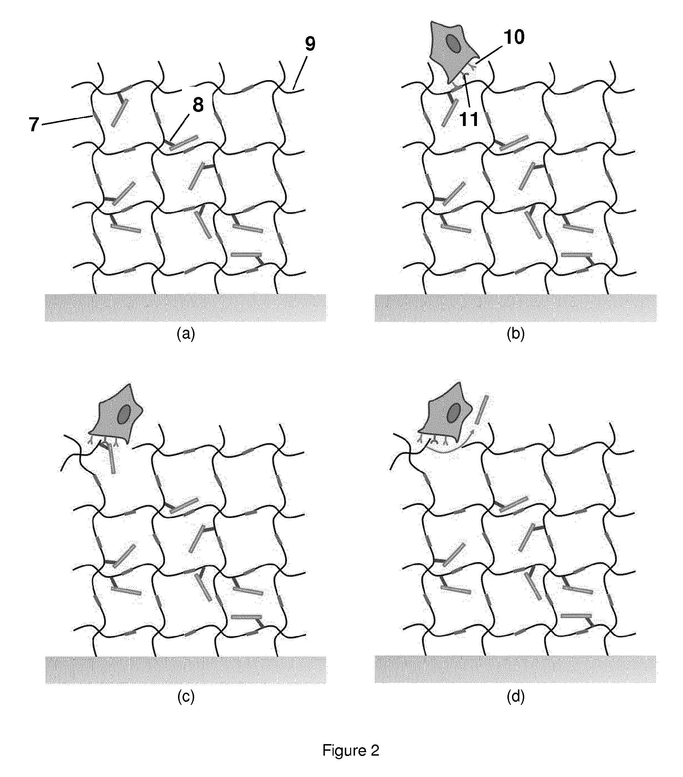

Figure 2 shows another possible embodiment of the invention. Here again, the multi-branched polymer chains (9) are linked together by peptide cross-linkers (7). In this construction of the coating, drug molecules are attached to the coating through another peptide cross-linker than the one used to link the multi-branched polymer chains. These two peptide chains may react to enzyme present on or released by the same cell (as in the example shown in figure 2) or they may react to enzyme present on or released by different cells. In the case described in figure 2, the cell will first cleave the peptide used to create the network (figure 2(b)) and then, after opening of the network, access to the drug molecule (figure 2(c)) and cleave its link to the polymer chain. In this way it will liberate the molecule (figure 2(d)). In the case of peptides reacting to two different types of cells, the first type will destroy the network, inducing its dissolution, while the other will liberate the drug molecules.

### PEG-based coatings

Taking PEG as an example of basis material that could be used to create such coatings, there are two common methods of forming PEG-based hydrogels: chain growth and step growth polymerization of multifunctional PEG monomers.

In chain growth, PEG di(meth)acrylate monomers (PEGDMA) are polymerized by free radical chain polymerization, forming coiled poly(methacrylate) chains connected by PEG linkers. The advantage of this approach where the reaction is photoinitiated is to allow the formation of a hydrogel under physiological and cytocompatible conditions. The resulting molecular mesh size of the network is dictated by the length of the PEG chain and the concentration of PEGDMA in the gel-forming solution. In addition, the crosslinking density of the network is controlled by the concentration of PEGDMA, dictating the resultant gel modulus, equilibrium water content, and solute diffusivity. Degradation is easily introduced within these gels by incorporation of a degradable moiety within the PEGDMA crosslinking monomer.

In step growth polymerization, multifunctional PEG monomers are reacted stoichiometrically with degradable linkers to form nearly perfect networks by step growth polymerization. The reactive end groups may include acrylate and thiols polymerized by base-catalysed Michael-type reaction, norbornenes and thiols by free radical initiation, and azide and alkynes by copper-based or copper-free click reaction. Here the mesh size is dictated by the length of the PEG chain, whereas the crosslinking density is dictated by the length and concentration of the PEG crosslinker.

A possible way to create a coating as described in this invention is to use on one side multibranched PEG chains functionalized with vinyl sulfone groups and on the other side Cys-oligopeptides. A tri-dimensional network will be formed via a conjugate addition reaction (called Michael type reaction). This network will have plastic properties defined by the type of the used PEG precursor (in particular the length of the chains) as well as by the conditions of gel formation (in particular the pH at which the reaction takes places). The active agent can be added to the precursor solution and thus be entrapped into the final network.

The table below gives non limitative examples of known enzyme - degradable sequence combinations. A major advantage of such sort of pairs is their high specificity.

| **Enzyme** | **Degradable sequence** |
|---|---|
| Lysosomal protease | GFLG |
| Collagenase | GGLGPAGGK |
| Elastase | AAAAAAAAAK; AAPVR; AAP(Nva) |
| Plasmin | VRN |
| Thrombin | G d-F Pipecolic Acid RSGGGGKC |
| Collagenase and various matrix metalloproteinases (MMPs), such as MMP-1, -2, -3, -7, -8 and -9 | GPQ**GI**WGQ; GPQGIAGQ; GPQGILGQ |
| MMP-2 and MMP-9 | PVGLIG; GPA**GL**GC |
| MMP-2 | GPLGIAGQ |

## Claims

1. A coating for an implantable medical device comprising a structural material and an active agent, said structural material and/or active agent containing a substance which, when brought into contact with a cell, induces the dissolution of said structural material and thereby allows the release of said active agent

2. A coating according to claim 1 wherein the type of cells able to induce the dissolution of the coating is limited and defined, and is specific to the coating and or said substance.

3. A coating according to any preceding claims wherein the dissolution of the coating takes place only in the presence of particular cells

4. A coating according to claim 1 to 3 wherein the active agent is:
• a toxic agent for the cells, including at least one compound of the list: paclitaxel, sirolimus, everolimus, tacrolimus, zotarolimus or biolimus; or
• a contrast medium; or
• an agent that favours cell proliferation.

5. A coating according to any preceding claims wherein the active agent is composed of different molecules

6. A coating according to any preceding claim wherein the structural material is made of a polymeric material

7. A coating according to claim 6 wherein the polymeric material is a hydrogel

8. A coating according to any preceding claims wherein the structural material is made of a combination of polymer coupled to peptides that are cleaved by enzymes released by cells or present at the surface of the cells

9. A coating according to claim 8 wherein the enzymes are azoreductase, tyrosine kinase, cathepsins, alkaline and acid phosphatase, matrix metalloproteinases, elastase, lipase, β-lactamase, thermolysin, plasmin, collagenase, urokinase, plasminogen-activator or factor Xa.

10. A coating according to any preceding claims wherein the active agent is covalently attached to the coating

11. A coating according to claim 6 wherein the active agent is coupled to the coating using a peptide

12. A coating according to claims 5 and 11 wherein different molecules are coupled to the coating with different peptides

13. A coating according to claims 1 to 9 wherein the active agent is dispersed within the coating material

14. A coating according to claim 13 wherein the active agent dispersed into the matrix is first encapsulated into a degradable structure or attached to a polymer chain, a branched polymer, a dendrimer, or a particle

15. A coating according to anyone of the preceding claim wherein an intermediate layer is created between the coating and the substrate to improve the adhesion of the coating onto the substrate.
